# EUROPÄISCHE PATENTSCHRIFT

(11) **EP 2 905 043 B1**
(45) Veröffentlichungstag und Bekanntmachung des Hinweises auf die Patenterteilung: **08.04.2020**
(21) Anmeldenummer: 15152298.4
(22) Anmeldetag: 23.01.2015
(51) Int. Cl.: A61M 5/142, A61M 5/20, A61M 5/315, A61M 5/32, A61M 39/10, A61M 5/34

(54) **Verbindungsmodul für eine Spritze und Spritze mit einem solchen Verbindungsmodul**
Connection module for a syringe and syringe with such a connection module
Module de liaison pour une seringue et seringue avec un tel module de liaison

(30) Priorität: 27.01.2014 DE 102014201392
(43) Veröffentlichungstag der Anmeldung: 12.08.2015
(73) Patentinhaber: Henke-Sass, Wolf GmbH, 78532 Tuttlingen (DE)
(72) Erfinder: Raidt, Simon, 78573 Wurmlingen (DE); Altermann, Frank, 78532 Tuttlingen (DE)
(74) Vertreter: Patentanwälte Geyer, Fehners & Partner mbB

(56) Entgegenhaltungen:
- EP-A1- 1 693 079
- WO-A1-2010/028040
- WO-A2-02/066100
- WO-A2-2011/029056
- JP-A- H07 148 271
- US-A1- 2008 255 542

## Beschreibung

Die Erfindung betrifft ein Verbindungsmodul für eine Spritze mit den Merkmalen des Oberbegriffs des Anspruchs 1. Solche Verbindungsmodule können z.B. als Luer-Lock-Verbindung ausgebildet sein und werden beispielsweise für medizinische Anwendungen eingesetzt, wobei das erste Verbindungsteil eine Kanüle oder ein Schlauch sein kann und das zweite Verbindungsteil beispielsweise Bestandteil einer Spritze sein kann.

Ferner betrifft die vorliegende Erfindung eine Spritze mit einem solchen Verbindungsmodul.

Bei einem solchen Verbindungsmodul besteht häufig die Schwierigkeit, dass ein Lösen der Verbindung schwierig ist. Wenn das erste Verbindungsteil als Kanüle ausgebildet ist, besteht immer eine Verletzungsgefahr. Insbesondere kann die Kanüle kontaminiert sein, was zu einer Infektionsgefahr führt.

Die WO 02/066100 A2 zeigt ein Verbindungsmodul mit den Merkmalen des Oberbegriffs des Anspruches 1.

Die WO 2010/028040 A1, WO 2011/029056 A2, US 2008/255542 A1, EP 1 693 079 A1, die JP 07-148271 A sowie die US 2007/265577 A1 zeigen weitere Verbindungsmodule mit einem ersten Verbindungsteil, das einen ersten Fluidkanal aufweist und einem zweiten Verbindungsteil, das einen zweiten Fluidkanal aufweist.

Ausgehend hiervon ist es daher Aufgabe der Erfindung, ein eingangs beschriebenes Verbindungsmodul so weiterzubilden, dass in sicherer Weise ein Lösen der Verbindung möglich ist.

Erfindungsgemäß wird die Aufgabe durch ein Verbindungsmodul mit den Merkmalen des Anspruches 1 gelöst.

Mit dem erfindungsgemäßen Verbindungsmodul ist somit leicht ein Lösen der Verbindung möglich. Es muß lediglich die Verschraubung gelöst werden und dies kann durch Betätigen der Aufnahmehülse erfolgen, so dass das erste Verbindungsteil, das z.B. eine Kanüle sein kann, nicht in die Hand genommen werden muß.

Das zweite Verbindungsmittel kann zwei, drei, vier oder mehr voneinander beabstandete Vorsprünge aufweisen, die jeweils in einer Richtung radial zum ersten Füllkanal vorstehen.

Eine der beiden Kontaktflächen kann als Innenkegelfläche und die andere der beiden Kontaktflächen kann als Außenkegelfläche ausgebildet sein.

Die Kanüle kann einen Sockelabschnitt aufweisen, an dem das erste Verbindungsmittel ausgebildet ist.

Insbesondere kann die Verbindung als Luer-Lock-Verbindung ausgebildet sein.

Die Aufnahmehülse kann als Aufnahmemutter ausgebildet sein, bei der der erste Gewindeabschnitt ein Innengewindeabschnitt ist.

Es wird ferner eine Spritze mit einem Grundkörper, der einen Spritzenzylinder mit einem ausspritzseitigen Ende aufweist, und einer Kolbenstange, deren vorderer Endabschnitt in dem Spritzenzylinder entlang einer Verschieberichtung verschiebbar angeordnet ist, bereitgestellt, bei der ein erfindungsgemäßes Verbindungsmodul vorgesehen ist, wobei das zweite Verbindungsteil Teil des Grundkörpers oder mit diesen verbunden ist und der zweite Fluidkanal in Fluidverbindung mit dem Spritzenzylinder steht.

Bei der erfindungsgemäßen Spritze kann der Grundkörper einen vorderen Anschlag für die Kolbenstange, bei der der Abstand zwischen dem vorderen Endabschnitt und dem ausspritzseitigen Ende des Spritzenzylinders minimal ist, aufweisen. Des weiteren kann die Spritze ein Einstellelement umfassen, das einen hinteren Anschlag für die Kolbenstange, bei der der Abstand zwischen dem vorderen Endabschnitt und dem ausspritzseitigen Ende des Spritzenzylinder maximal ist, aufweist, wobei das Einstellelement relativ zum Grundkörper bewegbar und in zumindest zwei vorgegebenen Einstellpositionen, in denen die Position des hinteren Anschlags in Verschieberichtung verschieden ist, lösbar mit dem Grundkörper verbindbar ist, so dass durch Wahl einer der Einstellpositionen für das Einstellelement der maximale Hub der Kolbenstange einstellbar ist.

Mit der erfindungsgemäßen Spritze kann somit in einfacher Art und Weise der maximale Kolbenstangenhub und somit die Ausspritzmenge eingestellt werden.

Damit kann wiederholt in sicherer Art und Weise die eingestellte Ausspritzmenge appliziert werden.

Insbesondere kann die Spritze als Selbstfüllerspritze ausgebildet sein. Unter einer Selbstfüllerspritze wird insbesondere eine Spritze verstanden, bei der das zu applizierende Fluid (z.B. Medikament) bei einer Bewegung der Kolbenstange vom vorderen Anschlag zum hinteren Anschlag in den Spritzenzylinder gefüllt wird und bei der entgegengesetzten Bewegung vom hinteren Anschlag zum vorderen Anschlag über das ausspritzseitige Ende aus dem Spritzenzylinder ausgespritzt wird.

Bei der erfindungsgemäßen Spritze kann das Einstellelement entlang der Verschieberichtung relativ zum Grundkörper bewegbar sein.

Insbesondere kann das Einstellelement hohlzylinderförmig mit einem Boden ausgebildet sein, wobei der Boden den hinteren Anschlag bildet.

Der Boden kann eine Durchgangsbohrung für die Kolbenstange aufweisen. Die Kolbenstange kann einen seitlichen Vorsprung (z.B. ringförmig) aufweisen, der an einer Innenseite des Bodens anliegt, wenn die Kolbenstange am hinteren Anschlag ist. Der Kontakt zwischen dem Vorsprung und der Innenseite des Bodens bildet somit den hinteren Anschlag.

Die Kolbenstange kann einstückig oder auch mehrstückig sein. Der Bereich der Kolbenstange, der im Spritzenzylinder hin und her bewegt wird, kann auch als Kolben oder Kolbenabschnitt bezeichnet werden.

Das Einstellelement kann ein federndes Druckstück aufweisen und der Grundkörper kann in jeder vorgegebenen Einstellposition eine Rastvertiefung für das federnde Druckstück aufweisen.

Insbesondere kann der Grundkörper eine Führungsrille oder auch Führungsnut für das federnde Druckstück aufweisen, wobei sich die Führungsrille bzw. -nut entlang der Verschieberichtung erstreckt und das federnde Druckstück beim Verschieben des Einstellelements relativ zum Grundkörper entlang der Verschieberichtung führt.

Bei der erfindungsgemäßen Spritze kann für jede vorgegebene Einstellposition eine Justierrille (bzw. Justiernut) von der Führungsrille quer zur Führungsrille abzweigen, in die das federnde Druckstück durch ein relatives Verdrehen von Einstellelement und Grundkörper bringbar ist. Insbesondere weist jede Justierrille eine Rastvertiefung auf, so dass in Zusammenwirkung mit dem federnden Druckstück die gewünschte Arretierung erzeugt wird.

Diese Arretierung kann durch ein entgegengesetztes Verdrehen gelöst werden, wenn die aufgewandte Kraft so groß ist, dass das federnde Druckstück aus der Rastvertiefung gelöst wird.

Anstatt eines federnden Druckstücks sind auch andere Fixierelemente möglich. Beispielsweise kann ein Verklemmen durch eine Schraube durchgeführt werden, die im Einstellelement geführt ist und gegen den Grundkörper drückt.

Bei der erfindungsgemäßen Spritze kann die Position des vorderen Anschlags nicht änderbar sein. Dies ist insbesondere bei der Ausbildung als Selbstfüllerspritze sehr vorteilhaft, da das Befüllen des Spritzenzylinders über das ausspritzseitige Ende durchführbar ist und stets das gesamte Fluid aus dem Spritzenzylinder ausgespritzt werden kann.

Die erfindungsgemäße Spritze kann auch als Injektionsvorrichtung und/oder Injektionssystem bezeichnet werden. Weitere Elemente, die für den Betrieb eines solchen Injektionssystems notwendig sind, sind dem Fachmann bekannt und können vorgesehen sein. Insbesondere kann die Kolbenstange z.B. pneumatisch von einem Druckluftzylinder angetrieben werden. Dazu kann das vom Spritzenzylinder wegweisende Ende der Kolbenstange mit dem Druckluftzylinder mechanisch verbunden sein.

Die erfindungsgemäße Spritze kann eine Kanüle aufweisen, die mit dem ausspritzseitigen Ende des Spritzenzylinders in Fluidverbindung steht, so dass das Fluid aus dem Spritzenzylinder über die Kanüle applizierbar ist.

Die Kanüle kann lösbar am Grundkörper befestigt sein. Insbesondere kann die lösbare Verbindung so ausgebildet sein, dass zum Lösen der Kanüle diese nicht in die Hand genommen werden muß.

Dazu kann die Kanüle einen ersten Fluidkanal und eine erste Kontaktfläche aufweisen. Des weiteren kann der Grundkörper der Spritze ein Verbindungsteil mit einem zweiten Fluidkanal und einer zweiten Kontaktfläche aufweisen. Das Verbindungsteil kann durch einen Abschnitt des Grundkörpers selbst oder als separates, mit dem Grundkörper verbundenes Teil ausgebildet sein.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine perspektivische Ansicht einer ersten Ausführungsform der erfindungsgemäßen Spritze;
- Fig. 2: eine perspektivische Explosionsdarstellung der Spritze 1 von Fig. 1;
- Fig. 3: eine Schnittdarstellung gemäß der Schnittlinie A-A in Fig. 2;
- Fig. 4: eine Längsschnittdarstellung der erfindungsgemäßen Spritze 1, bei der der hintere Anschlag 21 in einer ersten vorgegebenen Einstellposition ist;
- Fig. 5: eine Schnittdarstellung gemäß Fig. 4, bei der der hintere Anschlag 21 in einer zweiten vorgegebenen Einstellposition ist;
- Fig. 6: eine Schnittdarstellung gemäß Fig. 5 einer Abwandlung der erfindungsgemäßen Spritze 1;
- Fig. 7: eine Draufsicht der Mutter 54 der erfindungsgemäßen Spritze 1;
- Fig. 8: eine Schnittansicht der Aufnahmemutter 54 entlang der Schnittlinie B-B in Fig. 7;
- Fig. 9: eine Schnittansicht der Aufnahmemutter 54 entlang der Schnittlinie C-C in Fig. 7;
- Fig. 10: eine Draufsicht der Kanüle 13;
- Fig. 11: eine vergrößerte Schnittdarstellung der Kanüle 13, der Aufnahmemutter 54 und des Adapters 12 der erfindungsgemäßen Spritze 1, und
- Fig. 12: eine Schnittansicht gemäß Fig. 11, wobei die Kanüle 13 um ihre Längsachse um 90° verdreht ist gegenüber der Position gemäß Fig. 11.

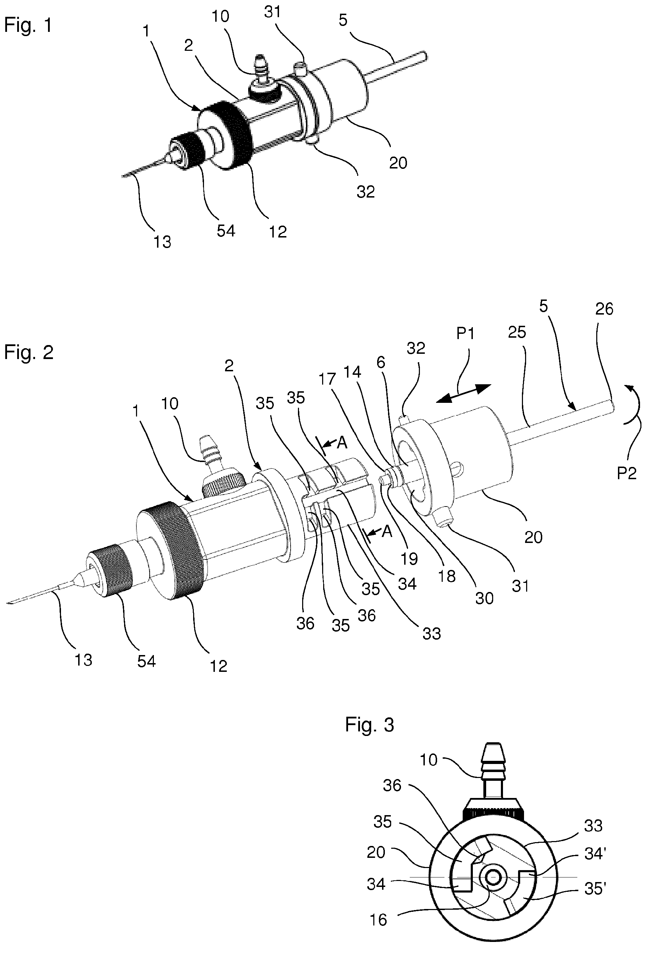

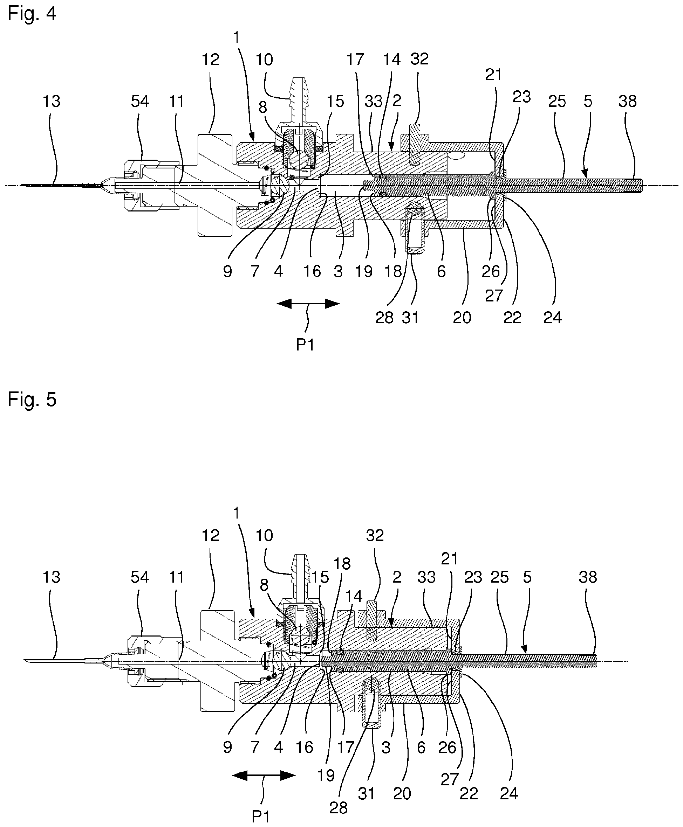

Bei der in Fig. 1-5 gezeigten Ausführungsform umfaßt die erfindungsgemäße Spritze 1 einen Grundkörper 2, in dem ein Spritzenzylinder 3 mit einem ausspritzseitigen Ende 4 (Fig. 4 und 5) ausgebildet ist.

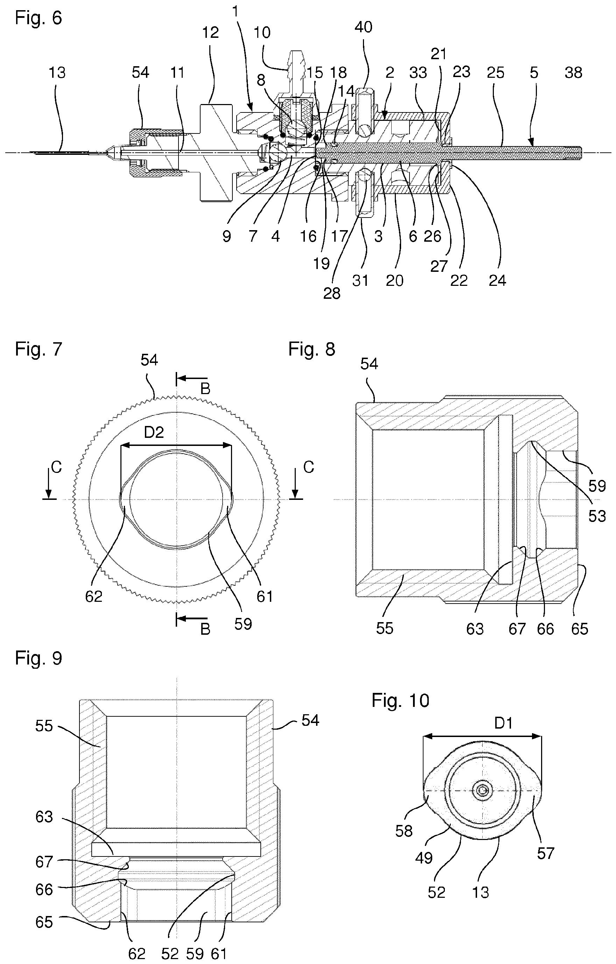

Ferner umfaßt die Spritze 1 eine Kolbenstange 5, deren vorderer Endabschnitt 6 im Spritzenzylinder 3 entlang einer Verschieberichtung P1 (in Fig. 5 und 6 von links nach rechts) verschiebbar angeordnet ist.

Das ausspritzseitige Ende 4 des Spritzenzylinders 3 mündet im Grundkörper 2 in einem T-förmigen Kanalabschnitt 7, in dessen Enden, die nicht mit dem ausspritzseitigen Ende 4 verbunden sind, ein erstes und zweites Rückschlagventil 8, 9 angeordnet sind. Das erste Rückschlagventil 8 ist so ausgebildet, dass bei einem Zurückziehen der Kolbenstange 5 (also einer Bewegung von links nach rechts in Fig. 4 und 5) ein Fluid, wie z.B. ein zu applizierendes Medikament, über einen Fluidanschluß 10 und das erste Rückschlagventil 8 in den Spritzenzylinder 3 fließt bzw. gesaugt wird. Das Fluid kann insbesondere eine Flüssigkeit sein. Das zweite Rückschlagventil 9 ist so ausgebildet, dass bei einer entgegengesetzten Bewegung der Kolbenstange 5 (also von rechts nach links in Fig. 4 und 5) das im Spritzzylinder 3 befindliche Fluid über das zweite Rückschlagventil 9 in einen Kanal 11 eines Adapters 12 und von diesem durch eine mit dem Adapter 12 verbundene Kanüle 13 ausgespritzt wird.

Der Adapter 12 und die Kanüle 13 bilden zusammen mit einer Aufnahmemutter ein erfindungsgemäßes Verbindungsmodul, das nachfolgend in Verbindung mit Fig. 7-12 detaillierter beschrieben wird. Zunächst werden jedoch andere Elemente der Spritze 1 beschrieben.

Die erfindungsgemäße Spritze 1 ist so ausgebildet, dass der maximale Hub der Kolbenstange 5 und somit die Menge des abzugebenden Fluids einstellbar ist. Dazu weist der Grundkörper 2 einen vorderen Anschlag 15 für die Kolbenstange 5 auf, der durch Schultern 16 am ausspritzseitigen Ende 4 des Spritzenzylinders 3 gebildet ist. Die Schultern 16 sind dadurch gebildet, dass der Durchmesser des Spritzenzylinders 3 größer ist als der Durchmesser des anschließenden Kanalteils des T-förmigen Kanalabschnitts 7.

Der vordere Endabschnitt 6 der Kolbenstange 5 weist an seinem vorderen Ende 17 eine ringförmige Anschlagfläche 18 auf, die an den Schultern 16 anliegt, wenn der vordere Endabschnitt 6 vollständig in den Spritzenzylinder 3 eingeschoben ist. Bei der hier beschriebenen Ausführungsform weist das vordere Ende 17 noch einen zylinderförmigen Vorsprung 19 auf, der dann in den entsprechenden Teil des T-förmigen Kanalabschnittes 7 einsteht. In einer alternativen Ausführungsform (nicht gezeigt) ist der Vorsprung 19 nicht vorgesehen, so dass das vordere Ende 17 z.B. plan ausgebildet sein kann und in seinem äußeren Bereich die ringförmige Anschlagfläche 18 aufweist. Ferner weist die Kolbenstange 5 im Bereich des vorderen Endabschnitts 6 einen Dichtring 14 auf, der in einer entsprechenden Ringnut sitzt. Wenn die ringförmige Anschlagfläche 18 an den Schultern 16 anliegt, ist der Abstand zwischen dem vorderen Ende 17 und dem ausspritzseitigen Ende 4 minimal.

Die Spritze 1 weist ferner ein hülsenförmiges Einstellelement 20 auf, das einen hinteren Anschlag 21 für die Kolbenstange 5 festlegt, bei dem der Abstand zwischen dem vorderen Ende 17 und dem ausspritzseitigen Ende 4 des Spritzenzylinders 3 maximal ist. Dazu ist im Boden 22 des einen U-förmigen Querschnitt aufweisenden Einstellelementes 20 eine Durchgangsbohrung 23 ausgebildet, durch die die Kolbenstange 5 hindurchläuft. Bei der hier beschriebenen Ausführungsform ist in der Durchgangsbohrung 23 eine Führungshülse 24 eingesetzt, die eine Öffnung im Boden 22 definiert, die koaxial zum Spritzenzylinder 3 ist und dabei einen geringeren Durchmesser als der Spritzenzylinder 3 aufweist.

Die Kolbenstange 5 ist so ausgebildet, dass der vordere Endabschnitt 6 einen an den Spritzenzylinder 3 angepaßten Außendurchmesser aufweist und somit als Kolben dient. An den vorderen Endabschnitt 6 schließt sich ein hinterer Abschnitt 25 der Kolbenstange 5 an, der einen geringeren Durchmesser als der vordere Endabschnitt 6 aufweist. Der Durchmesser des hinteren Abschnitts 25 ist an die durch die Führungshülse 24 definierte Öffnung angepaßt. Ferner weist der vordere Endabschnitt 6 an seinem dem vorderen Ende 17 abgewandten Ende, an das der hintere Abschnitt 25 der Kolbenstange 3 anschließt, einen im wesentlichen ringförmigen Vorsprung 26 auf, der in den Querschnittsdarstellungen gemäß Fig. 4 und 5 an einer Innenseite 27 des Bodens 22 anliegt. Die Innenseite 27 bildet somit den hinteren Anschlag 21 für die Kolbenstange 5, da ein Herausziehen der Kolbenstange 5 aus dem Spritzenzylinder 3 über diese Position nicht möglich ist.

Das Einstellelement 20 ist, wie nachfolgend im Detail beschrieben wird, in Verschieberichtung P1 der Kolbenstange 5 relativ zum Grundkörper 2 verschiebbar und an vorgegebenen Einstellpositionen mit dem Grundkörper 2 fixier- bzw. arretierbar, so dass dadurch die Position des hinteren Anschlags 21 in Verschiebrichtung P1 einstellbar und veränderbar ist. Bei der erfindungsgemäßen Spritze 1 ist der vordere Anschlag 15 fixiert bzw. ist seine Position nicht veränderbar, während der hinter Anschlag 21 verstellbar ist. Mit dieser Art der Einstellung der Position des hinteren Anschlages 21 wird der maximale Hub der Kolbenstange 5 und somit die maximale Fluidmenge festgelegt, die mit einer einzelnen Kolbenstangenbewegung (Verschieben der Kolbenstange 5 von ihrer hinteren Anschlagposition zu ihrer vorderen Anschlagposition) ausspritzbar ist.

Wie in Fig. 4 und 5 ersichtlich ist, weist das Einstellelement 20 im Bereich seines vorderen offenen Endes 30 ein federndes Druckstück 31 und einen Stift 32 auf, die sich jeweils in radialer Richtung erstrecken und einander gegenüberliegend angeordnet sind. Das federnde Druckstück 31 weist an seinem nach innen vorstehenden Ende eine Kugel 28 auf, die durch eine nicht gezeigte Feder mit einer Kraft in Richtung zum Spritzenzylinder 3 hin beaufschlagt ist und zur Arretierung des Einstellelementes 20 dient. Der Stift 32 und das federnde Druckstück 31 sind so im vorderen offenen Ende 30 angeordnet, dass sie über eine Innenwandung des Einstellelementes 20 nach innen vorstehen. Der Durchmesser der Innenwandung des Einstellelementes 20 entspricht dem Außendurchmesser des Grundkörpers 2 in seinem hinteren Endabschnitt 33, in dem der Spritzenzylinder 3 ausgebildet ist. Um eine Verschiebbarkeit des Einstellelementes 20 relativ zum Grundkörper 2 bzw. auf dem hinteren Endabschnitt 33 des Grundkörpers 2 zu ermöglichen, ist sowohl für das federnde Druckstück 31 als auch für den Stift 32 eine sich in Verschieberichtung erstreckende Führungsrille 34, 34' auf der Außenseite des hinteren Endabschnitts 33 ausgebildet. In der perspektivischen Explosionsdarstellung gemäß Fig. 2 ist die Führungsrille 34 für das federnde Druckstück 31 ersichtlich. In der Schnittdarstellung in Fig. 3 (Schnitt entlang A-A in Fig. 2) sind beide Führungsrillen 34, 34' erkennbar. Von der Führungsrille 34 sowie von der Führungsrille 34' gehen jeweils vier Justierrillen 35, 35' ab, die sich jeweils in Umfangsrichtung des hinteren Endabschnittes 33 entlang eines vorbestimmten Winkelbereiches erstrecken. Die Justierrillen 35 für das federnde Druckstück 31 enden jeweils in einer Rastvertiefung 36. Bei dem hier beschriebenen Ausführungsbeispiel erstrecken sich zwei der vier Justierrillen 35 im Uhrzeigersinn und die anderen zwei Justierrillen 35 entgegengesetzt zum Uhrzeigersinn. Gleiches trifft auf die Justierrillen 35' zu. In Verschieberichtung (Doppelpfeil P1 in Fig. 2, 4 und 5) sind die vier Justierrillen 35 und die vier Justierrillen 35' jeweils voneinander beabstandet, so dass vier vorgegebene Einstellpositionen vorhanden sind. Die in Fig. 2 nicht sichtbare Führungsrille 34' für den Stift 32 sowie die entsprechenden Justierrillen 35' weisen jedoch aufgrund des geringeren Durchmessers des Stifts 32 im Vergleich zum federnden Druckstück 31 eine geringere Breite aufweisen. Ferner enden die Justierrillen 35' für den Stift 32 nicht in einer Rastvertiefung.

In der Schnittansicht gemäß Fig. 3 ist eine Justierrille 35 gezeigt, die mit einer Rastvertiefung 36 endet. Die entsprechende Justierrille 35' für den Stift 32 endet ohne Rastvertiefung.

Zur Einstellung eines gewünschten maximalen Kolbenstangenhubs ist ausgehend von der Explosionsdarstellung in Fig. 2 das Einstellelement 20 um 90° gegen den Uhrzeigersinn (Drehrichtung gemäß Pfeil P2 in Fig. 2) zu drehen, so dass das nach innen vorstehende Ende (mit der Kugel 28) des federnden Druckstücks 31 entlang der Führungsrille 34 bewegt werden kann. Das Einstellelement 20 ist dann entlang der Verschieberichtung P1 so zu bewegen, dass das federnde Druckstück 31 auf der Höhe der gewünschten Justierrille 35 liegt und dann wird das Einstellelement 20 durch Drehung in Richtung zu der Rastvertiefung 36 der gewählten Justierrille 35 (also entweder im Uhrzeigersinn oder entgegen des Uhrzeigersinns) arretiert, da bei Erreichen der Rastvertiefung 36 die Kugel 28 des federnden Druckstücks 31 in die Rastvertiefung 36 gedrückt wird.

Somit kann erfindungsgemäß der maximale Kolbenstangenhub eingestellt werden. Die Spritze 1 weist somit verschiedene Rastpositionen für den hinteren Anschlag 21 auf.

Bei der hier beschriebenen Ausführungsform wird die Kolbenstange 5 pneumatisch von einem nicht gezeigten Druckluftzylinder angetrieben. Die entsprechende Verbindung kann mit dem von dem Grundkörper 2 weg weisenden Ende 38 des hinteren Abschnitts 25 der Kolbenstange 5 erfolgen. Bei der beschriebenen Ausführungsform ist am Ende 38 der Kolbenstange 5 ein Außengewinde ausgebildet.

In Fig. 6 ist eine Schnittansicht gemäß Fig. 4 und 5 in einer Abwandlung der erfindungsgemäßen Spritze 1 gezeigt. Die Spritze 1 gemäß Fig. 6 unterscheidet sich von der bisher beschriebenen Spritze 1 darin, dass anstatt des Stiftes 32 ein zweites federndes Druckstück 40 vorgesehen ist. Die entsprechende Führungsrille 34' sowie die entsprechenden Justierrillen 35' sind daher in gleicher Weise wie für das federnde Druckstück 31 der bisher beschriebenen Ausführungsform ausgebildet. Insbesondere weisen die Justierrillen 35' für das zweite federnde Druckstück 40 jeweils eine Rastvertiefung auf. Die restliche Ausbildung der Spritze 1 gemäß Fig. 6 entspricht der Ausbildung der Spritze der Fig. 1 bis 5, so dass gleiche Elemente mit gleichen Bezugszeichen bezeichnet sind und zu deren Beschreibung auf die obigen Ausführungen verwiesen wird.

Bei den Ausführungsformen gemäß Fig. 1 bis 6 ist die Kanüle 13 lösbar am Adapter 12 befestigt, wobei der Adapter 12 lösbar (über eine Schraubverbindung) mit dem Grundkörper 2 verbunden ist. Die Verbindung zwischen Kanüle 13 und Adapter 12 wird nachfolgend in Verbindung mit Fig. 7-12 beschrieben und ist als sogenannte Luer-Lock-Verbindung ausgebildet, wobei der Adapter 12 an seinem dem Spritzenzylinder 3 abgewandten Ende einen Außenkegelabschnitt 50 aufweist, auf dem ein entsprechender Innenkegelabschnitt 51 eines Sockels 52 der Kanüle 13 sitzt. Ein ringförmiger Vorsprung 49 des Sockels 52 der Kanüle 13 sitzt in einer ringförmigen Haltenut 53 einer Aufnahmemutter 54, die auf den Adapter 12 aufgeschraubt ist. Dazu weist die Aufnahmemutter 54 einen Innengewindeabschnitt 55 auf und ist am Adapter 12 ein entsprechender Außengewindeabschnitt 56 ausgebildet.

Wie insbesondere der Draufsicht der Kanüle 13 in Fig. 10 zu entnehmen ist, ist der Umriß des ringförmigen Vorsprungs 49 des Fußes 52 im wesentlichen kreisförmig und weist zwei gegenüberliegende seitliche Vorsprünge 57, 58 auf, so dass dadurch eine Abweichung von der Kreisform vorliegt.

Die Haltenut 53 ist an einer Innenseite einer Durchgangsbohrung 59 im Boden 60 der im Querschnitt im wesentlichen U-förmigen Aufnahmemutter 54 ausgebildet (Fig. 8, 9, 11, 12). Der Bereich der Durchgangsbohrung 59, der von dem dem Adapter 12 abgewandten vorderen Ende 65 der Aufnahmemutter 54 bis zur Haltenut 53 verläuft, weist im Querschnitt einen Umriß auf, der dem Umriß des ringförmigen Vorsprungs 49 der Kanüle 13 entspricht. Wie insbesondere in der Draufsicht von Fig. 7 ersichtlich ist, ist der Umriß der Durchgangsbohrung 59 im wesentlichen kreisförmig und weist zwei radial nach außen vorstehende Ausnehmungen 61, 62 auf. Diese Ausnehmungen 61 und 62 der Durchgangsbohrung 59 erstrecken sich jedoch nur vom vorderen Ende 65 bis zur Haltenut 53. Im Bereich von der Haltenut 53 bis zur dem Adapter 12 zugewandten Innenseite 63 des Bodens 60 sind die Ausnehmungen 61 und 62 nicht ausgebildet, so dass hier die Durchgangsbohrung 59 lediglich eine kreisförmige Querschnittsform aufweist.

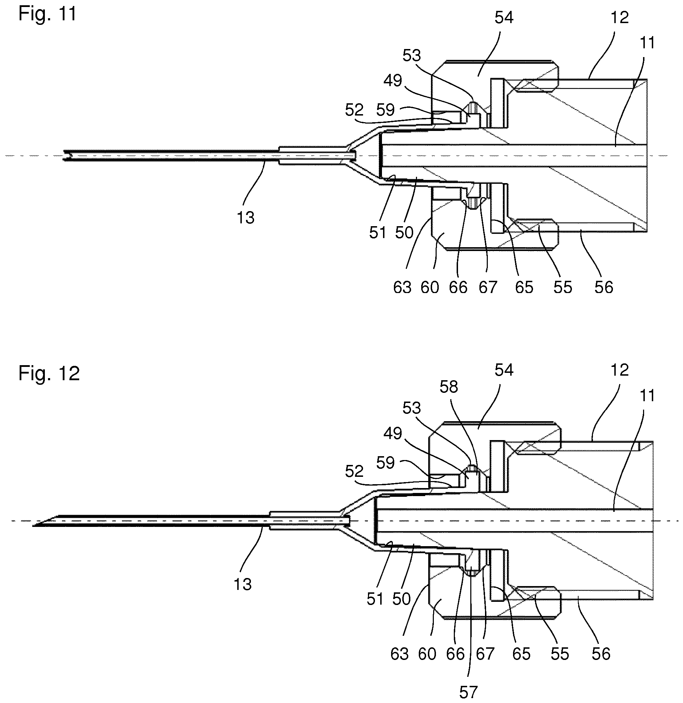

Die Durchgangsbohrung 59 mit den Ausnehmungen 61 und 62 ist so ausgebildet, dass der ringförmige Vorsprung 49 der Kanüle 13 vom vorderen Ende 65 der Aufnahmemutter 54 bis zur Haltenut 53 eingeführt werden kann. Sobald der ringförmige Vorsprung 49 so in die Haltenut 53 eingeführt ist (Fig. 11), kann die Kanüle 13 relativ zur Aufnahmemutter 54 um die Längsachse der Kanüle 13 gedreht werden, wodurch die Vorsprünge 57 und 58 in einem Bereich in der Haltenut 53 positioniert werden, in dem keine Ausnehmungen 61 und 62 ausgebildet sind, so dass die Kanüle 13 in der Haltenut 53 sitzt (Fig. 12).

Bevorzugt ist die maximale Ausdehnung D1 des ringförmigen Vorsprungs, die durch die beiden Vorsprünge 57 und 58 festgelegt ist, so gewählt, dass sie etwas größer ist als die maximale, durch die Ausnehmungen 61, 62 vorhandene lichte Weite D2 der Durchgangsbohrung 59. In diesem Fall muß die Kanüle 13 zum Einsetzen in die Aufnahmemutter 54 leicht gekippt werden (gegenüber der Zeichenebene von Fig. 7). Damit ist es möglich, trotz der größeren Ausdehnung D1 der Vorsprünge 57 und 58 den ringförmigen Vorsprung 49 der Kanüle 13 durch die Ausnehmungen 61, 62 bis zur Haltenut 53 in die Aufnahmemutter 54 einzuführen. Die Dicke der Haltenut 53 (Ausdehnung von links nach rechts in Fig. 8) ist dabei so gewählt, dass der Sockel 52 und somit auch der ringförmige Vorsprung 49 der Kanüle 13 in der Haltenut 53 wieder zurück gekippt werden kann (es liegt somit ein ausreichendes Spiel zwischen dem ringförmigen Vorsprung 49 und der Haltenut 53 vor), so dass selbst ohne Verdrehen der Kanüle 13 relativ zur Aufnahmemutter 54 schon eine gewisse Haltefunktion durch die Haltenut 53 bereitgestellt wird.

Beim Aufschrauben der Aufnahmemutter 54 mit eingesetzter Kanüle 13 liegen die Vorsprünge 57 und 58 an der vorderen Seite 66 der Haltenut 53 an, so dass dadurch der Innenkegelabschnitt 51 der Kanüle 13 auf den Außenkegelabschnitt 50 des Adapters 12 gedrückt wird. Durch den flächigen Kontakt der beiden Kegelabschnitte 50, 51 wird eine Dichtwirkung erreicht. Somit wird die gewünschte Fluidabdichtung zwischen dem Kanal 11 und der Kanüle 13 realisiert, und ein bestimmungsgemäßer Gebrauch der Spritze 1 ist möglich.

Wenn die Kanüle 13 ausgetauscht werden soll, muß lediglich die Aufnahmemutter 54 abgeschraubt werden. Bei diesem Abschrauben drückt dann die hintere Seite 67 der Haltenut 53 gegen die Vorsprünge 57, 58 des Sockels bzw. Fußes 52 der Kanüle 13 und zieht somit automatisch die Kanüle 13 von dem Innenkegelabschnitt 50 des Adapters 12 ab. Es ist somit ein Lösen der Kanüle 13 in sicherer Art und Weise möglich, da die Kanüle 13 selbst nicht in die Hand genommen werden muß. Allein durch Abschrauben der Aufnahmemutter 54 wird die Kanüle 13 vom Adapter 12 gelöst.

Das erfindungsgemäße Verbindungsmodul (Kanüle 13, Adapter 12 und Aufnahmemutter 54) wurde hier nur beispielshalber in Verbindung mit der Spritze 1 beschrieben. Bei dem erfindungsgemäßen Verbindungsmodul kann anstatt einer Kanüle 13 auch ein sonstiges Element mit einem Fluidkanal, das einen Sockel 52 in der beschriebenen Art und Weise aufweist, verwendet werden. Beispielsweise kann ein Schlauch in dieser Art und Weise angeschlossen werden. Auch muß der Adapter 12 kein separates Element sein, sondern kann Bestandteil des Grundkörpers 2 sein oder kann auch durch ein anderes Element mit einem Fluidkanal 11 ersetzt werden. Wesentlich ist hier, dass eine lösbare Verbindung zwischen den beiden zu verbindenden Verbindungsteilen 13, 12 vorliegt, bei der lediglich durch Abschrauben der Aufnahmemutter 54 ein Lösen der Verbindung bewirkt wird.

## Patentansprüche

1. Verbindungsmodul für eine Spritze, mit
einem ersten Verbindungsteil (13), das einen ersten Fluidkanal und eine erste Kontaktfläche (51) aufweist, einem zweiten Verbindungsteil (12), das einen zweiten Fluidkanal (11) und eine zweite Kontaktfläche (50) aufweist,
wobei eine der beiden Kontaktflächen (51) als Innenfläche und die andere der beiden Kontaktflächen als Außenfläche (50), die komplementär zur Innenfläche (51) ist, ausgebildet ist, wobei das Verbindungsmodul ferner eine Aufnahmehülse (54) mit einem ersten Gewindeabschnitt (55) und einem ersten Verbindungsmittel (53) für das erste Verbindungsteil (13) umfaßt,
das erste Verbindungsteil (13) ein zweites Verbindungsmittel (57, 58) aufweist, das mit dem ersten Verbindungsmittel (53) so zusammenwirkt, dass das erste Verbindungsteil (13) lösbar mit der Aufnahmehülse (54) verbunden ist,
wobei das zweite Verbindungsteil (12) einen zum ersten Gewindeabschnitt (55) komplementären zweiten Gewindeabschnitt (56) aufweist und
durch Verschrauben der beiden Gewindeabschnitte (55, 56) die beiden Fluidkanäle (11) miteinander verbunden sind sowie die erste Kontaktfläche (51) des ersten Verbindungsteils (13) dichtend in Kontakt mit der zweiten Kontaktfläche (50) des zweiten Verbindungsteils (12) gebracht ist,
und wobei bei Lösen der Verschraubung der beiden Gewindeabschnitte (55, 56) die Aufnahmehülse (54) über die beiden Verbindungsmittel (53, 57, 58) eine Kraft auf das erste Verbindungsteil (13) ausübt, die die erste Kontaktfläche (51) von der zweiten Kontaktfläche (50) weg bewegt,
wobei eines der beiden Verbindungsmittel (51, 57, 58) eine Haltenut (53) und das andere der beiden Verbindungsmittel (53, 57, 58) einen Vorsprung (57, 58) aufweist, der im verschraubten Zustand in die Haltenut (53) einsteht,
**dadurch gekennzeichnet,**
**dass** die Aufnahmehülse (54) eine Durchgangsbohrung (59) mit einer Innenwandung aufweist, wobei das erste Verbindungsmittel die Haltenut (53) aufweist, die in der Innenwandung ausgebildet ist,
**dass** das zweite Verbindungsmittel (57, 58) zwei voneinander beabstandete Vorsprünge (57, 58) aufweist, die jeweils in einer Richtung radial zum ersten Fluidkanal vorstehen,
und **dass** die Aufnahmehülse (54) an ihrem dem zweiten Verbindungsteil (12) abgewandten ersten Ende (65) für jeden Vorsprung (57, 58) eine Ausnehmung (61, 62) aufweist, die sich vom ersten Ende (65) bis zur Haltenut (53) erstrecken, so dass das erste Verbindungsteil (13) über die Ausnehmungen (61, 62) in die Haltenut (53) einsetzbar ist.

2. Verbindungsmodul nach Anspruch 1, bei dem eine der beiden Kontaktflächen (50, 51) als Innenkegelfläche (51) und die andere der beiden Kontaktflächen (50, 51) als Außenkegelfläche (50) ausgebildet ist.

3. Verbindungsmodul nach einem der obigen Ansprüche, bei dem das erste Verbindungsteil (13) eine Kanüle mit einem Sockel (52) umfaßt und das erste Verbindungsmittel am Sockel (52) ausgebildet ist.

4. Verbindungsmodul nach einem der obigen Ansprüche, wobei das Verbindungsmodul als Luer-Lock-Anschluss ausgebildet ist.

5. Verbindungsmodul nach einem der obigen Ansprüche, wobei die Aufnahmehülse (54) als Aufnahmemutter (54) ausgebildet ist, bei der der erste Gewindeabschnitt (55) als Innengewindeabschnitt ausgebildet ist.

6. Spritze mit
einem Grundkörper (2), der einen Spritzenzylinder (3) mit einem aufspritzseitigen Ende (4) aufweist, und
einer Kolbenstange (5), deren vorderer Endabschnitt (6) in dem Spritzenzylinder (3) entlang einer Verschieberichtung (P1) verschiebbar angeordnet ist, wobei die Spritze ein Verbindungsmodul nach einem der obigen Ansprüche aufweist und das zweite Verbindungsteil, dessen zweiter Fluidkanal in Fluidverbindung mit dem Spritzenzylinder steht, Bestandteil der Spritze ist.

## Claims

1. Connecting module for a syringe with
a first connecting component (13), which has a first fluid channel and a first contact surface (51), a second connecting component (12), which has a second fluid channel (11) and a second contact surface (50),
wherein one of the two contact surfaces (51) is formed as an inner surface and the other of the two contact surfaces is formed as an outer surface (50) which is complementary to the inner surface (51), wherein the connecting module further comprises a receiving sleeve (54) with a first threaded section (55) and a first connecting device (53) for the first connecting component (13),
the first connecting component (13) has a second connecting device (57, 58), which interacts with the first connecting device (53) in such a way that the first connecting component (13) is detachably connected to the receiving sleeve (54),
wherein the second connecting component (12) has a second threaded section (56) which is complementary to the first threaded section (55) and
by screwing the two threaded sections (55, 56), the two fluid channels (11) are connected to each other and the first contact surface (51) of the first connecting component (13) is brought into contact in a sealing manner with the second contact surface (50) of the second connecting component (12), and wherein on unscrewing the two threaded sections (55, 56), the receiving sleeve (54) exerts a force via the two connecting devices (53, 57, 58) to the first connecting component (13) which moves the first contact surface (51) away from the second contact surface (50),
wherein one of the two connecting devices (51, 57, 58) has a retaining groove (53) and the other of the two connecting devices (53, 57, 58) has a projection (57, 58) which, in the screwed-in state, projects into the retaining groove (53),
**characterized in that**
the receiving sleeve (54) has a through hole (59) with an inner wall, wherein the first connecting device has the retaining groove (53) which is formed in the inner wall,
the second connecting device (57, 58) has two projections (57, 58) spaced apart from each other, which project in each case in a direction radial to the first fluid channel,
and the receiving sleeve (54), at its first end (65) facing away from the second connecting component (12), has, for each projection (57, 58), a recess (61, 62) which extends from the first end (65) to the retaining groove (53), with the result that the first connecting component (13) can be inserted via the recesses (61, 62) into the retaining groove (53).

2. Connecting module according to claim 1, in which one of the two contact surfaces (50, 51) is formed as an inner tapered surface (51) and the other of the two contact surfaces (50, 51) is formed as an outer tapered surface (50).

3. Connecting module according to any of the above claims, in which the first connecting component (13) comprises a cannula with a base (52) and the first connecting device is formed on the base (52).

4. Connecting module according to any of the above claims, wherein the connecting module is formed as a Luer lock connection.

5. Connecting module according to any of the above claims, wherein the receiving sleeve (54) is formed as a receiving nut (54) in which the first threaded section (55) is formed as an internal threaded section.

6. Syringe with
a base body (2) which has a syringe cylinder (3) with an expelling side end (4), and
a plunger rod (5), the front end section (6) of which is arranged to be displaceable in the syringe cylinder (3) along a displacement direction (P1), wherein the syringe has a connecting module according to any of the above claims and the second connecting component, the second fluid channel of which is in fluid connection with the syringe cylinder, is part of the syringe.

## Revendications

1. Module de liaison pour une seringue, comprenant une première partie de liaison (13), qui possède un premier canal à fluide et une première surface de contact (51), une deuxième partie de liaison (12), qui possède un deuxième canal à fluide (11) et une deuxième surface de contact (50),
l'une des deux surfaces de contact (51) étant réalisée sous la forme d'une surface intérieure et l'autre des deux surfaces de contact sous la forme d'une surface extérieure (50), qui est complémentaire à la surface intérieure (51),
le module de liaison comportant en outre une douille d'accueil (54) comprenant une première portion filetée (55) et un premier moyen de liaison (53) pour la première partie de liaison (13),
la première partie de liaison (13) possédant un deuxième moyen de liaison (57, 58) qui coopère avec le premier moyen de liaison (53) de telle sorte que la première partie de liaison (13) est reliée de manière amovible à la douille d'accueil (54),
la deuxième partie de liaison (12) possédant une deuxième portion filetée (56) complémentaire à la première portion filetée (55) et
les deux canaux à fluide (11) étant reliés ensemble par vissage des deux portions filetées (55, 56) et la première surface de contact (51) de la première partie de liaison (13) étant amenée en contact étanche avec la deuxième surface de contact (50) de la deuxième partie de liaison (12),
et lors du desserrage du vissage des deux portions filetées (55, 56), la douille d'accueil (54) exerçant par le biais des deux moyens de liaison (53, 57, 58) sur la première partie de liaison (13) une force qui écarte la première surface de contact (51) de la deuxième surface de contact (50),
l'un des deux moyens de liaison (51, 57, 58) possédant une rainure de maintien (53) et l'autre des deux moyens de liaison (53, 57, 58) une partie saillante (57, 58) qui, à l'état vissé, pénètre dans la rainure de maintien (53),
**caractérisé en ce**
**que** la douille d'accueil (54) possède un alésage traversant (59) muni d'une paroi interne, le premier moyen de liaison possédant la rainure de maintien (53) qui est formée dans la paroi interne,
**que** le deuxième moyen de liaison (57, 58) possède deux parties saillantes (57, 58) espacées l'une de l'autre, qui font respectivement saillie dans une direction radiale par rapport au premier canal à fluide,
et **que** la douille d'accueil (54) possède pour chaque partie saillante (57, 58), au niveau de sa première extrémité (65) qui est opposée à la deuxième partie de liaison (12), une cavité (61, 62) qui s'étend de la première extrémité (65) jusqu'à la rainure de maintien (53), de sorte que la première partie de liaison (13) peut être introduite dans la rainure de maintien (53) par le biais des cavité (61, 62).

2. Module de liaison selon la revendication 1, dans lequel l'une des deux surfaces de contact (50, 51) est réalisée sous la forme d'une surface conique interne (51) et l'autre des deux surfaces de contact (50, 51) sous la forme d'une surface conique externe (50).

3. Module de liaison selon l'une des revendications précédentes, dans lequel la première partie de liaison (13) comporte une canule munie d'un socle (52) et le premier moyen de liaison est formé au niveau du socle (52) .

4. Module de liaison selon l'une des revendications précédentes, le module de liaison étant réalisé sous la forme d'un raccordement de type Luer-Lock.

5. Module de liaison selon l'une des revendications précédentes, la douille d'accueil (54) étant réalisée sous la forme d'un écrou d'accueil (54) avec lequel la première portion filetée (55) est réalisée sous la forme d'une portion filetée femelle.

6. Seringue comprenant
un corps de base (2) qui possède un cylindre de seringue (3) ayant une extrémité (4) côté injection et
une tige de piston (5) dont la portion d'extrémité avant (6) est disposée coulissante dans le cylindre de seringue (3) le long d'une direction de coulissement (P1), la seringue possédant un module de liaison selon l'une des revendications précédentes et la deuxième partie de liaison, dont le deuxième canal à fluide se trouve en liaison fluidique avec le cylindre de seringue, étant un élément constitutif de la seringue.
